# EUROPÄISCHE PATENTSCHRIFT

(11) **EP 1 204 651 B1**
(45) Veröffentlichungstag und Bekanntmachung des Hinweises auf die Patenterteilung: **20.09.2006**
(21) Anmeldenummer: 00951397.9
(22) Anmeldetag: 18.07.2000
(51) Int. Cl.: C07D 285/13, A01N 43/824

(54) **HERBIZIDE 5-CHLORDIFLUORMETHYL-1, 3, 4-THIADIAZOL-2-YL-OXYACETANILIDE**
HERBICIDAL 5-CHLORODIFLUOROMETHYL-1,3,4-THIADIAZOL-2-YL-OXYACETANILIDES
5-CHLORODIFLUOROMETHYL-1,3-4-THIADIAZOL-2YL-OXYACETANILIDES HERBICIDES

(30) Priorität: 30.07.1999 DE 19935964
(43) Veröffentlichungstag der Anmeldung: 15.05.2002
(73) Patentinhaber: Bayer CropScience AG, 40789 Monheim (DE)
(72) Erfinder: RIEBEL, Hans-Jochem, D-56242 Selters (DE); FÖRSTER, Heinz, D-56337 Kadenbach (DE); DREWES, Mark, Wilhelm, D-40764 Langenfeld (DE); DAHMEN, Peter, D-41470 Neuss (DE); FEUCHT, Dieter, D-40789 Monheim (DE); PONTZEN, Rolf, D-42799 Leichlingen (DE)
(74) Vertreter: Schwenk, Norbert
(86) Internationale Anmeldenummer: PCT/EP2000/006851
(87) Internationale Veröffentlichungsnummer: WO 2001/009109

(56) Entgegenhaltungen:
- EP-A- 0 148 501
- EP-A- 0 348 737
- EP-A- 0 626 380

## Beschreibung

Die Erfindung betrifft 5-Chlordifluormethyl-1,3,4-thiadiazol-2-yl-oxyacetanilide, ein Verfahren zu ihrer Herstellung und ihre Verwendung als Herbizide.

Es ist bereits bekannt, daß bestimmte 5-Chlordifluormethyl-1,3,4-thiadiazol-2-yl-oxyacetanilide wie z.B. die Verbindungen N-Methyl-5-chlordifluormethyl-1,3,4-thiadiazol-2-yl-oxyacetanilid, N-Ethyl-5-chlordifluormethyl-1,3,4-thiadiazol-2-yl-oxyacetanilid, 4-Chlor-N-methyl-(5-chlordifluormethyl-1,3,4-thiadiazol-2-yl)-oxyacetanilid und 3-Trifluormethyl-N-methyl-(5-chlordifluormethyl-1,3,4-thiadiazol-2-yl)-oxyacetanilid (vgl.EP-A-148501 / US-A-4798731), die Verbindung N-i-Propyl-5-chlordifluormethyl-1,3,4-thiadiazol-2-yl-oxyacetanilid (vgl. EP-A-348737 / US-A-4968342) sowie die Verbindungen 4-Fluor-N-methyl-(5-chlordifluormethyl-1,3,4-thiadiazol-2-yl)-oxyacetanilid und 4-Fluor-N-ethyl-(5-chlordifluormethyl-1,3,4-thiadiazol-2-yl)-oxyacetanilid herbizide Eigenschaften aufweisen (vgl. EP-A-626380). Die Wirkung dieser vorbekannten Verbindungen ist jedoch, insbesondere bei niedrigen Aufwandmengen und Konzentrationen, nicht in allen Anwendungsgebieten völlig zufriedenstellend.

Es wurden nun 5-Chlordifluormethyl-1,3,4-thiadiazol-2-yl-oxyacetanilide der allgemeinen Formel (I) 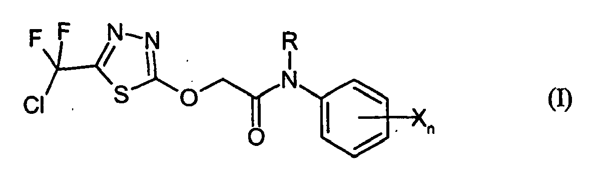 in welcher
- R: für i-Propyl, s- oder t-Butyl steht,
- n: für 1 oder 2, und
für n = 1 (d.h. X verschieden von H); X für (2) CN, (3) CN, (4) CN, (2) F, (3) F, (4) F, (2) Cl, (3) Cl, (4) Cl, (2) CH₃, (3) CH₃, (4) CH₃, (2) C₂H₅, (3) C₂H₅, (4) C₂H₅, (2) CF₃, (3) CF₃, (4) CF₃, (2) Br, (3) Br, (4) Br, (2) OCH₃, (3) OCH₃, (4) OCH₃, (2) OCF₃, (3) OCF₃, (4) OCF₃ steht, oder
für n = 2 (d.h. X verschieden von H); X in zwei Positionen (mit gleicher oder verschiedener Bedeutung) für (2,3) F₂, (2,4) F₂, (2,5) F₂, (3,4) F₂, (3,5) F₂, (2) F / (3) Cl, (2) F / (4) Cl, (2) F / (5) Cl, (3) F / (4) Cl, (2) F / (3) Br, (2) F / (4) Br, (2) F / (5) Br, (2) F / (3) CH₃, (2) F / (4) CH₃, (2) F / (5) CH₃, (3) F / (4) CH₃, (2) F / (3) C₂H₅, (2) F / (4) C₂H₅, (2) F / (5) C₂H₅, (2) F / (3) CF₃, (2) F /(4) CF₃, (2) F / (5) CF₃, (2,3) Cl₂, (2,4) Cl₂, (2,5) Cl₂, (3,4) Cl₂, (2) Cl / (3) F, (2) Cl / (4) F, (2) Cl / (5) F, (3) Cl / (4) F, (2) Cl / (3) Br, (2) Cl / (4) Br, (2) Cl / (5) Br, (2) Cl / (3) CH₃, (2) Cl / (4) CH₃, (2) Cl / (5) CH₃, (2) Cl / (3) C₂H₅, (2) Cl / (4) C₂H₅, (2) Cl / (5) C₂H₅, (2) Cl / (3) CF₃, (2) Cl / (4) CF₃, (2) Cl / (5) CF₃, (3) Cl / (4) CF₃, (2) Br / (3) F, (2) Br / (4) F, (2) Br / (5) F, (2) Br / (3) Cl, (2) Br / (4) Cl, (2) Br / (5) Cl, (2) Br / (3) CH₃, (2) Br / (4) CH₃, (2) Br / (5) CH₃, (2) Br / (3) CF₃, (2) Br / (4) CF₃, (2) Br / (5) CF₃, (2,3) (CH₃)₂, (2,4) (CH₃)₂, (2,5) (CH₃)₂, (3,4) (CH₃)₂, (2) CH₃ / (3) F, (2) CH₃ / (4) F, (2) CH₃ / (5) F, (3) CH₃ / (4) F, (2) CH₃ / (3) Cl, (2) CH₃ / (4) Cl, (2) CH₃ / (5) Cl, (2) CH₃ / (3) Br, (2) CH₃ / (4) Br, (2) CH₃ / (5) Br, (2) CH₃ / (3) CF₃, (2) CH₃ / (4) CF₃, (2) CH₃ / (5) CF₃, (2) C₂H₅ / (3) F, (2) C₂H₅ / (4) F, (2) C₂H₅ / (5) F, (2,3) (CF₃)₂, (2,4) (CF₃)₂, (2,5) (CF₃)₂, (2) CF₃ / (3) F, (2) CF₃ / (4) F, (2) CF₃ / (5) F, (2) CF₃ / (3) Cl, (2) CF₃ / (4) Cl, (2) CF₃ / (5) Cl, (2) Cl / (4) OCH₃, (2) Cl / (5) OCH₃, (2) F / (4) OCH₃, (2) OCF₃ / (4) F, (2) OCF₃ / (4) Cl, (2) F / (4) OCF₃ oder (2) Cl / (4) OCF₃ steht,
gefunden.

Angegeben sind die Bedeutungen von Xₙ und (in Klammern vorangestellt) Positionen von Xₙ.

Die oben aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Gegebenenfalls substituierte Reste können einfach oder mehrfach substituiert sein, wobei bei Mehrfachsubstitution die Substituenten gleich oder verschieden sein können.

Beispiele für die erfindungsgemäßen Verbindungen der allgemeinen Formel (I) sind in den nachstehenden Gruppen aufgeführt.

### Gruppe 1

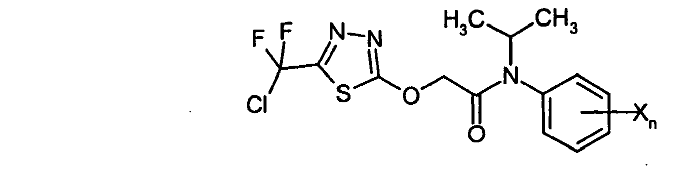

Xₙ hat hierbei die oben angegebene Bedeutung.

### Gruppe 2

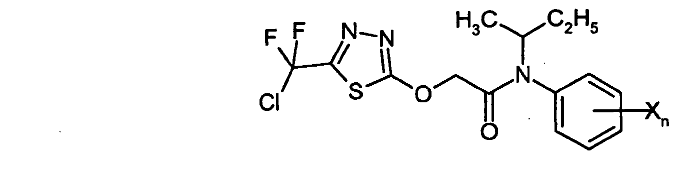

Xₙ hat hierbei die oben angegebene Bedeutung.

### Gruppe 3

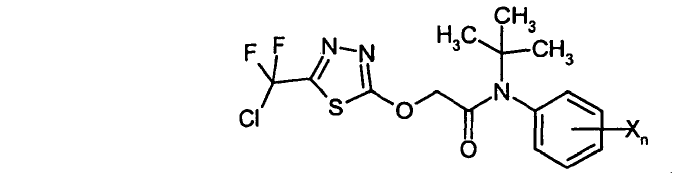

Xₙ hat hierbei die oben angegebene Bedeutung.

Erfindungsgemäß bevorzugt ist die Verbindungen der Formel

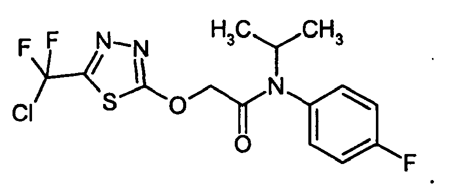

Die 5-Chlordifluormethyl-1,3,4-thiadiazol-2-yl-oxyacetanilide der allgemeinen Formel (I) weisen interessante biologische Eigenschaften auf. Sie zeichnen sich insbesondere durch starke und selektive herbizide Wirksamkeit aus.

Man erhält die 5-Chlordifluormethyl-1,3,4-thiadiazol-2-yl-oxyacetanilide der allgemeinen Formel (I), wenn man
5-Chlordifluormethyl-2-methylsulfonyl-1,3,4-thiadiazol der Formel (II) 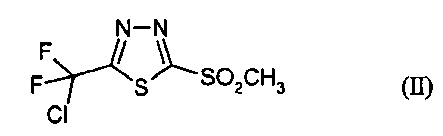 mit Hydroxyacetaniliden der allgemeinen Formel (III) 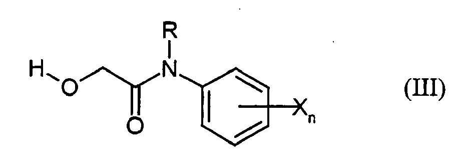 in welcher
n, R und X die oben angegebene Bedeutung haben,
gegebenenfalls in Gegenwart eines Säurebindemittels und gegebenenfalls in Gegenwart eines Verdünnungsmittels umsetzt.

Verwendet. man beispielsweise 5-Chlordifluormethyl-2-methylsulfonyl-1,3,4-thiadiazol und N-(t-Butyl)-N-(3-fluor-phenyl)-2-hydroxyacetamid als Ausgangsstoffe, so kann der Reaktionsablauf beim erfindungsgemäßen Verfahren durch das folgende Formelschema skizziert werden:

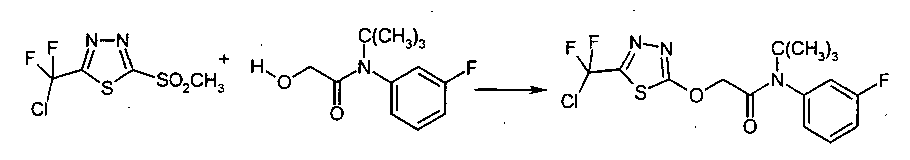

Die beim erfindungsgemäßen Verfahren zur Herstellung von Verbindungen der allgemeinen Formel (I) als Ausgangsstoff zu verwendende Verbindung 5-Chlordifluormethyl-2-methylsulfonyl-1,3,4-thiadiazol der Formel (II) ist bereits bekannt (vgl. EP-A-298338).

Man erhält die Verbindung 5-Chlordifluormethyl-2-methylsulfonyl-1,3,4-thiadiazol der Formel (II), wenn man beispielsweise in einer ersten Stufe Chlordifluoressigsäure (ClF₂C-COOH) mit Dithiocarbazidsäure-methylester (NH₂NH-C(S)-S-CH₃) in Gegenwart von Phosphorylchlorid (P(O)Cl₃) bei Temperaturen zwischen 0°C und 100°C umsetzt und die hierbei gebildete Verbindung 5-Chlordifluormethyl-2-methyl-thio-1,3,4-thiadiazol mit einem Oxidationsmittel, wie z.B. Hydrogenperoxid (H₂O₂), gegebenenfalls in Gegenwart eines Katalysators, wie z.B. Natriumwolframat, und gegebenenfalls in Gegenwart eines Verdünnungsmittels, wie z.B. Essigsäure, bei Temperaturen zwischen 0°C und 50°C umsetzt (vgl. die Herstellungsbeispiele).

Die beim erfindungsgemäßen Verfahren zur Herstellung von Verbindungen der allgemeinen Formel (I) als Ausgangsstoffe zu verwendenden Hydroxyacetanilide sind durch die Formel (III) allgemein definiert. In der allgemeinen Formel (III) haben n, R und X vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für n, R und X angegeben worden sind.

Die Ausgangsstoffe der allgemeinen Formel (III) sind bekannt und/oder können nach an sich bekannten Verfahren hergestellt werden (vgl. EP-A-18749, EP-A-148501, EP-A-165537, EP-A-308740, EP-A-348735, EP-A-348737, EP-A-626380).

Als Verdünnungsmittel zur Durchführung des erfindungsgemäßen Verfahrens zur Herstellung der Verbindungen der allgemeinen Formel (I) kommen neben Wasser vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Als Säurebindemittel für das erfindungsgemäße Verfahren kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium-, -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8-Diazabicyclo[5,4,0]-undec-7-en (DBU).

Die Reaktionstemperaturen können bei der Durchführung des erfindungsgemäßen Verfahrens in einem größeren Bereich variiert werden. Im allgemeinen arbeitet man bei Temperaturen zwischen -20°C und 100°C, vorzugsweise zwischen 0°C und 60°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Säurebindemittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise
Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzobicyclon, Benzofenap, Benzoylprop(-ethyl), Bialaphos, Bifenox, Bispyribac(-sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlornitrofen, Chlorsulfuron, Chlortoluron, Cinidon(-ethyl), Cinmethylin, Cinosulfuron, Clefoxydim, Clethodim, Clodinafop(-propargyl), Clomazone, Clomeprop, Clopyralid, Clopyrasulfuron(-methyl), Cloransulam(-methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron(-methyl), Ethofumesate, Ethoxyfen, Ethoxysulfuron, Etobenzanid, Fenoxaprop-(-P-ethyl), Fentrazamide, Flamprop(-isopropyl), Flamprop(-isopropyl-L), Flamprop(-methyl), Flazasulfuron, Florasulam, Fluazifop(-P-butyl), Fluazolate, Flucarbazone, Flufenacet, Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop-(-ethoxyethyl), Haloxyfop(-P-methyl), Hexazinone, Imazamethabenz(-methyl), Imazamethapyr, Imazamox, Imazapic, Imazapyr, Imazaquin, Imazethapyr, Imazosulfuron, Iodosulfuron(-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop-(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele

### Beispiel 1

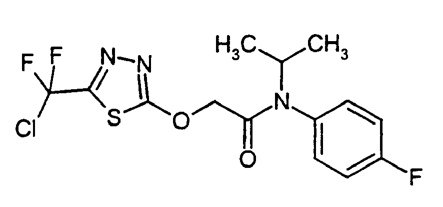

Eine Mischung aus 10 g (47 mMol) N-i-Propyl-N-(4-fluor-phenyl)-2-hydroxy-acetamid, 11,8 g (47 mMol) 5-Chlordifluormethyl-2-methylsulfonyl-1,3,4-thiadiazol und 50 ml Aceton wird auf -20°C abgekühlt und bei dieser Temperatur unter Rühren mit einer Lösung von 3 g (75 mMol) Natriumhydroxid in 8 ml Wasser tropfenweise versetzt. Die Reaktionsmischung wird 25 Stunden bei -10°C gerührt, dann mit Wasser auf etwa das dreifache Volumen verdünnt und mit Methylenchlorid extrahiert. Die organische Phase wird mit Wasser gewaschen, mit Natriumsulfat getrocknet und filtriert. Das Filtrat wird im Wasserstrahlvakuum eingeengt, der Rückstand mit Ligroin digeriert und das kristallin angefallene Produkt durch Absaugen isoliert.

Man erhält 10 g (56% der Theorie) N-i-Propyl-N-(4-fluor-phenyl)-2-(5-chlordifluormethyl-1,3,4-thiadiazol-2-yl-oxy)-acetamid vom Schmelzpunkt 98°C.

Analog zu Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der allgemeinen Formel (I) hergestellt werden.

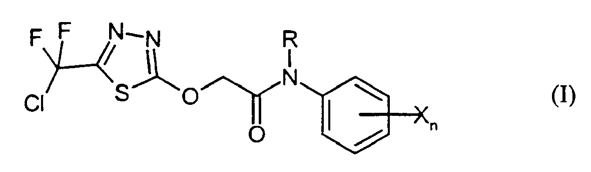

**Tabelle 1 Beispiele für die Verbindungen der Formel (I)**

| Bsp.-Nr. | R | (Position) Xₙ | Physikal. Daten |
|---|---|---|---|
| 2 | i-C₃H₇ | (4) Cl | Fp.: 78°C |
| 3 | i-C₃H₇ | (3) Cl | Fp.: 71°C |
| 4 | i-C₃H₇ | (3) CH₃ | Fp.: 57°C |
| 5 | i-C₃H₇ | (4) CH₃ | Fp.: 78°C |
| 6 | i-C₃H₇ | (3) OCH₃ | Fp.: 79°C |
| 7 | i-C₃H₇ | (3) F | Fp.: 60°C |
| 8 | i-C₃H₇ | (2,4) F₂ | Fp.: 84°C |

### Ausgangsverbindug der Formel (II)

### Beispiel II-1

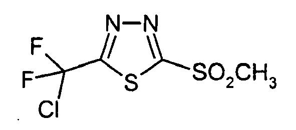

### Stufe 1

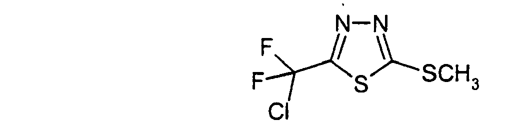

393 g (3 Mol) Chlordifluoressigsäure werden mit 372 g (3 Mol) Dithiocarbazidsäure-methylester vermischt. Dann werden 1000 g (6,54 Mol) Phosphorylchlorid innerhalb von zwei Stunden zu dieser Mischung tropfenweise gegeben wobei Gasentwicklung einsetzt. Die Reaktionsmischung wird dann langsam auf 70°C bis 80°C erhitzt und etwa 3 Stunden bei dieser Temperatur gehalten, wobei die Gasentwicklung allmählich abklingt. Anschließend wird die Mischung auf ca. 3 kg Eis gegeben und stehen gelassen, bis das überschüssige Phosphorylchlorid weitgehend zersetzt ist. Dann wird mit Chloroform geschüttelt, die organische Phase abgetrennt, mit Natriumsulfat getrocknet und filtriert. Das Filtrat wird im Wasserstrahlvakuum eingeengt und der Rückstand durch Destillation unter vermindertem Druck aufgearbeitet.

Man erhält 564 g (87% der Theorie) 5-Chlordifluormethyl-2-methylthio-1,3,4-thiadiazol vom Siedepunkt 62°C (bei 0,2 mbar).

### Stufe 2

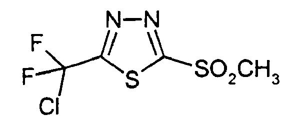

49,5 g einer 35%igen wässrigen Hydrogenperoxid-Lösung (0,57 Mol H₂O₂) werden innerhalb von zwei Stunden tropfenweise unter Rühren zu einer Mischung aus 22 g (0,10 Mol) 5-Chlordifluormethyl-2-methylthio-1,3,4-thiadiazol, 1 g Natriwnwolframat und 70 ml Essigsäure gegeben, wobei die Reaktionstemperatur bei 20°C bis 25°C gehalten wird. Die Reaktionsmischung wird bei dieser Temperatur 20 Stunden gerührt und dann mit 150 ml Chloroform verrührt. Die organische Phase wird abgetrennt, mit Wasser gewaschen, mit Natriumsulfat getrocknet und filtriert. Vom Filtrat wird das Lösungsmittel unter vermindertem Druck sorgfältig abdestilliert.

Man erhält 22,5 g (91% der Theorie) 5-Chlordifluormethyl-2-methylsulfonyl-1,3,4-thiadiazol vom Schmelzpunkt 46°C.

### Anwendungsbeispiele

### Beispiel A

**Pre-emergence-Test**

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden so mit der Wirkstoffzubereitung besprüht, daß die jeweils gewünschte Wirkstoffmenge pro Flächeneinheit ausgebracht wird. Die Wirkstoffkonzentration in der Spritzbrühe wird so gewählt, daß in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle. Es bedeuten:

| | |
|---|---|
| 0 % = | keine Wirkung (wie unbehandelte Kontrolle) |
| 100 % = | totale Vernichtung |

In diesem Test zeigt beispielsweise die Verbindung gemäß Herstellungsbeispiel 1 bei guter Verträglichkeit gegenüber Kulturpflanzen, wie z.B. Soja und Weizen, sehr starke Wirkung gegen Unkräuter.

**Tabelle A: Pre emergence-Test/Gewächshaus**

| Wirkstoff gemäß Herstellungsbeispiel-Nr. | Aufwandmenge (g ai./ha) | Weizen | Soja | Eriochloa | Lolium | Setaria | Galium | Matricaria |
|---|---|---|---|---|---|---|---|---|
| 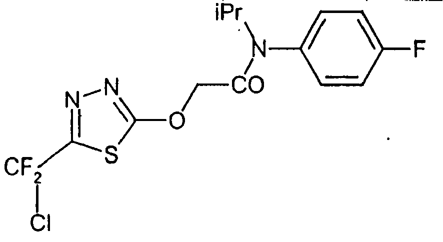 | | | | | | | | |
| (1) | 125 | 0 | 0 | 100 | 90 | 100 | 90 | 100 |

### Beispiel B

**Post-emergence-Test**

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigt beispielsweise die Verbindung gemäß Herstellungsbeispiel 1 bei guter Verträglichkeit gegenüber Kulturpflanzen, wie z.B. Mais, starke Wirkung gegen Unkräuter.

**Tabelle B Post emergence-Test/Gewächshaus**

| Wirkstoff gemäß Herstellungsbeispiel-Nr. | Aufwandmenge (g ai./ha) | Mais | Setaria | Galium | Xanthium |
|---|---|---|---|---|---|
| 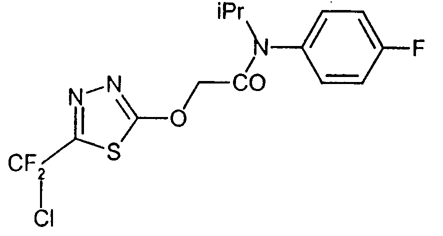 | | | | | |
| (1) | 1000 | 30 | 95 | 95 | 80 |

## Patentansprüche

1. Verbindungen der allgemeinen Formel (I), 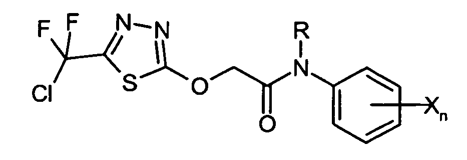 **dadurch gekennzeichnet, dass**
R für i-Propyl, s- oder t-Butyl steht,
n für 1 oder 2, und
für n = 1 (d.h. X verschieden von H);
X für (2) CN, (3) CN, (4) CN, (2) F, (3) F, (4) F, (2) Cl, (3) Cl, (4) Cl, (2) CH₃, (3) CH₃, (4) CH₃, (2) C₂H₅, (3) C₂H₅, (4) C₂H₅, (2) CF₃, (3) CF₃, (4) CF₃, (2) Br, (3) Br, (4) Br, (2) OCH₃, (3) OCH₃, (4) OCH₃, (2) OCF₃, (3) OCF₃, (4) OCF₃ steht, oder
für n = 2 (d.h. X verschieden von H);
X in zwei Positionen (mit gleicher oder verschiedener Bedeutung) für (2,3) F₂, (2,4) F₂, (2,5) F₂, (3,4) F₂, (3,5) F₂, (2) F / (3) Cl, (2) F / (4) Cl, (2) F / (5) Cl, (3) F / (4) Cl, (2) F / (3) Br, (2) F / (4) Br, (2) F / (5) Br, (2) F / (3) CH₃, (2) F / (4) CH₃, (2) F / (5) CH₃, (3) F / (4) CH₃, (2) F / (3) C₂H₅, (2) F / (4) C₂H₅, (2) F / (5) C₂H₅, (2) F / (3) CF₃, (2) F / (4) CF₃, (2) F / (5) CF₃, (2,3) Cl₂, (2,4) Cl₂, (2,5) Cl₂, (3,4) Cl₂, (2) Cl / (3) F, (2) Cl / (4) F, (2) Cl / (5) F, (3) Cl / (4) F, (2) Cl / (3) Br, (2) Cl / (4) Br, (2) Cl / (5) Br, (2) Cl / (3) CH₃, (2) Cl / (4) CH₃, (2) Cl / (5) CH₃, (2) Cl / (3) C₂H₅, (2) Cl / (4) C₂H₅, (2) Cl / (5) C₂H₅, (2) Cl / (3) CF₃, (2) Cl / (4) CF₃, (2) Cl / (5) CF₃, (3) Cl / (4) CF₃, (2) Br / (3) F, (2) Br / (4) F, (2) Br / (5) F, (2) Br / (3) Cl, (2) Br / (4) Cl, (2) Br / (5) Cl, (2) Br / (3) CH₃, (2) Br / (4) CH₃, (2) Br / (5) CH₃, (2) Br / (3) CF₃, (2) Br / (4) CF₃, (2) Br / (5) CF₃, (2,3) (CH₃)₂, (2,4) (CH₃)₂, (2,5) (CH₃)₂, (3,4) (CH₃)₂, (2) CH₃ / (3) F, (2) CH₃ / (4) F, (2) CH₃ / (5) F, (3) CH₃ / (4) F, (2) CH₃ / (3) Cl, (2) CH₃ / (4) Cl, (2) CH₃ / (5) Cl, (2) CH₃ / (3) Br, (2) CH₃ / (4) Br, (2) CH₃ / (5) Br, (2) CH₃ / (3) CF₃, (2) CH₃ / (4) CF₃, (2) CH₃ / (5) CF₃, (2) C₂H₅ / (3) F, (2) C₂H₅ / (4) F, (2) C₂H₅ / (5) F, (2,3) (CF₃)₂, (2,4) (CF₃)₂, (2,5) (CF₃)₂, (2) CF₃ / (3) F, (2) CF₃ / (4) F, (2) CF₃ / (5) F, (2) CF₃ / (3) Cl, (2) CF₃ / (4) Cl, (2) CF₃ / (5) Cl, (2) Cl / (4) OCH₃, (2) Cl / (5) OCH₃, (2) F / (4) OCH₃, (2) OCF₃ / (4) F, (2) OCF₃ / (4) Cl, (2) F / (4) OCF₃ oder (2) Cl / (4) OCF₃ steht.

2. Verbindung der Formel gemäß Anspruch 1

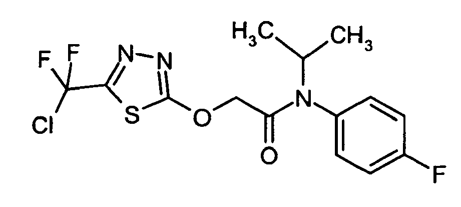

3. Verfahren zum Herstellen von Verbindungen gemäß Anspruch 1, **dadurch gekennzeichnet, dass**
5-Chlordifluormethyl-2-methylsulfonyl-1,3,4-thiadiazol der Formel (II) 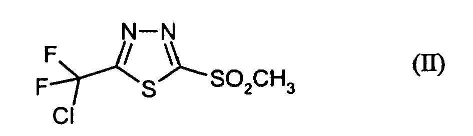 mit Hydroxyacetaniliden der allgemeinen Formel (III) 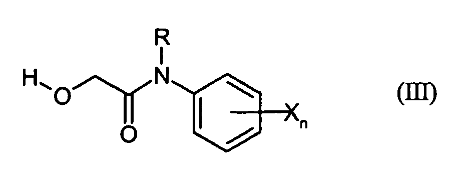 in welcher
n, R und X die in Anspruch 1 angegebene Bedeutung haben,
gegebenenfalls in Gegenwart eines Säurebindemittels und gegebenenfalls in Gegenwart eines Verdünnungsmittels umgesetzt werden.

4. Verfahren zum Bekämpfen von unerwünschtem Pflanzenwuchs, **dadurch gekennzeichnet, dass** man mindestens eine Verbindung gemäß einem der Ansprüche 1 bis 2 auf unerwünschte Pflanzen und/oder ihren Lebensraum einwirken lässt.

5. Verwendung von mindestens einer Verbindung gemäß einem der Ansprüche 1 bis 2 zum Bekämpfen von unerwünschten Pflanzen.

6. Herbizides Mittel, **gekennzeichnet durch** einen Gehalt an einer Verbindung gemäß einem der Ansprüche 1 bis 2 und üblichen Streckmitteln und/oder oberflächenaktiven Mitteln.

## Claims

1. Compounds of the general formula (I) 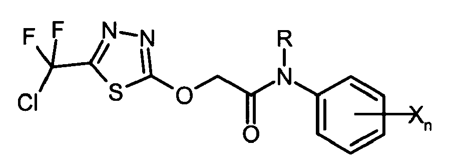 **characterized in that**
R represents i-propyl, s- or t-butyl,
n represents 1 or 2, and
for n = 1 (i.e. X different from H);
X represents (2) CN, (3) CN, (4) CN, (2) F, (3) F, (4) F, (2) Cl, (3) Cl, (4) Cl, (2) CH₃, (3) CH₃, (4) CH₃, (2) C₂H₅, (3) C₂H₅, (4) C₂H₅, (2) CF₃, (3) CF₃, (4) CF₃, (2) Br, (3) Br, (4) Br, (2) OCH₃, (3) OCH₃, (4) OCH₃, (2) OCF₃, (3) OCF₃, (4) OCF₃ or,
for n = 2 (i.e. X different from H);
X in two positions (with identical or different meaning) represents
(2,3) F₂, (2,4) F₂, (2,5) F₂, (3,4) F₂, (3,5) F₂, (2) F / (3) Cl, (2) F / (4) Cl, (2) F / (5) Cl, (3) F / (4) Cl, (2) F / (3) Br, (2) F / (4) Br, (2) F / (5) Br, (2) F / (3) CH₃, (2) F / (4) CH₃, (2) F / (5) CH₃, (3) F / (4) CH₃, (2) F / (3) C₂H₅, (2) F / (4) C₂H₅, (2) F / (5) C₂H₅, (2) F / (3) CF₃, (2) F / (4) CF₃, (2) F / (5) CF₃, (2,3) Cl₂, (2,4) Cl₂, (2,5) Cl₂, (3,4) Cl₂, (2) Cl / (3) F, (2) Cl / (4) F, (2) Cl / (5) F, (3) Cl / (4) F, (2) Cl / (3) Br, (2) Cl / (4) Br, (2) Cl / (5) Br, (2) Cl / (3) CH₃, (2) Cl / (4) CH₃, (2) Cl / (5) CH₃, (2) Cl / (3) C₂H₅, (2) Cl / (4) C₂H₅, (2) Cl / (5) C₂H₅, (2) Cl / (3) CF₃, (2) Cl / (4) CF₃, (2) Cl / (5) CF₃, (3) Cl /(4) CF₃, (2) Br / (3) F, (2) Br / (4) F, (2) Br / (5) F, (2) Br / (3) Cl, (2) Br / (4) Cl, (2) Br / (5) Cl, (2) Br / (3) CH₃, (2) Br / (4) CH₃, (2) Br / (5) CH₃, (2) Br / (3) CF₃, (2) Br / (4) CF₃, (2) Br / (5) CF₃, (2,3) (CH₃)₂, (2,4) (CH₃)₂, (2,5) (CH₃)₂, (3,4) (CH₃)₂, (2) CH₃ / (3) F, (2) CH₃ / (4) F, (2) CH₃ / (5) F, (3) CH₃ / (4) F, (2) CH₃ / (3) Cl, (2) CH₃ / (4) Cl, (2) CH₃ / (5) Cl, (2) CH₃ / (3) Br, (2) CH₃ / (4) Br, (2) CH₃ / (5) Br, (2) CH₃ / (3) CF₃, (2) CH₃ / (4) CF₃, (2) CH₃ / (5) CF₃, (2) C₂H₅ / (3) F, (2) C₂H₅ / (4) F, (2) C₂H₅ / (5) F, (2,3) (CF₃)₂, (2,4) (CF₃)₂, (2,5) (CF₃)₂, (2) CF₃ / (3) F, (2) CF₃ / (4) F, (2) CF₃ / (5) F, (2) CF₃ / (3) Cl, (2) CF₃ / (4) Cl, (2) CF₃ / (5) Cl, (2) Cl / (4) OCH₃, (2) Cl / (5) OCH₃, (2) F / (4) OCH₃, (2) OCF₃ / (4) F, (2) OCF₃ / (4) Cl, (2) F / (4) OCF₃ or (2) Cl / (4) OCF₃

2. Compound of the formula according to Claim 1

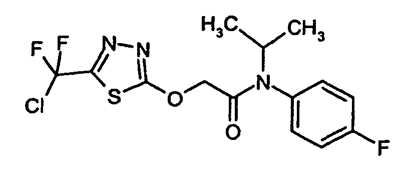

3. Process for preparing compounds according to Claim 1, **characterized in that**
5-chlorodifluoromethyl-2-methylsulphonyl-1,3,4-thiadiazole of the formula (II) 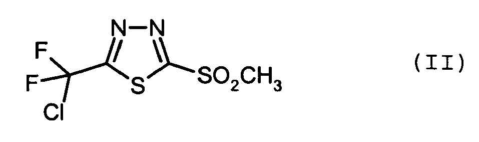 is reacted with hydroxyacetanilides of the general formula (III) 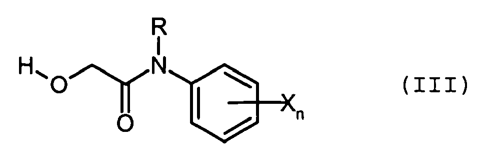 in which
n, R and X are each as defined in Claim 1,
if appropriate in the presence of an acid binder and if appropriate in the presence of a diluent.

4. Method for controlling undesirable vegetation, **characterized in that** at least one compound according to either of Claims 1 and 2 is allowed to act on undesirable plants and/or their habitat.

5. Use of at least one compound according to either of Claims 1 and 2 for controlling undesirable plants.

6. Herbicidal composition, **characterized in that** it comprises a compound according to either of Claims 1 and 2 and customary extenders and/or surfactants.

## Revendications

1. Composés de formule générale (I) 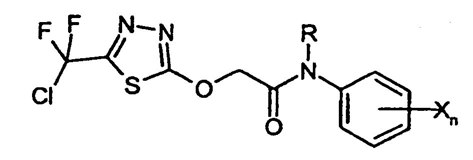 **caractérisés en ce**
R représente un groupe i-propyle, s- ou t-butyle,
n vaut 1 ou 2, et
pour n = 1 (c'est-à-dire différent de H) ;
X représente pour (2) CN, (3) CN, (4) CN, (2) F, (3) F, (4) F, (2) Cl, (3) Cl, (4) Cl, (2) CH₃, (3) CH₃, (4) CH₃, (2) C₂H₅, (3) C₂H₅, (4) C₂H₅, (2) CF₃, (3) CF₃, (4) CF₃, (2) Br, (3) Br, (4) Br, (2) OCH₃, (3) OCH₃, (4) OCH₃, (2) OCF₃, (3) OCF₃, (4) OCF₃ ou
pour n = 2 (c'est-à-dire différent de H) ;
x représente en deux positions (ayant une signification identique ou différente) pour (2,3) F₂, (2,4) F₂, (2,5) F₂, (3,4) F₂, (3,5) F₂, (2) F/(3) Cl, (2) F/(4) Cl, (2) F/(5) Cl, (3) F/(4) Cl, (2) F/(3) Br, (2) F/(4) Br, (2) F/(5) Br, (2) F/(3) CH₃, (2) F/(4) CH₃, (2) F/(5) CH₃, (3) F/(4) CH₃, (2) F/(3) C₂H₅, (2) F/(4) C₂H₅, (2) F/(5) C₂H₅, (2) F/(3) CF₃, (2) F/(4) CF₃, (2) F/(5) CF₃, (2,3) Cl₂, (2,4) Cl₂, (2,5) Cl₂, (3,4) Cl₂, (2) Cl/(3) F, (2) Cl/(4) F, (2) Cl/(5) F, (3) Cl/(4) F, (2) Cl/(3) Br, (2) Cl/(4) Br, (2) Cl/(5) Br, (2) Cl/(3) CH₃, (2) Cl/(4) CH₃, (2) Cl/(5) CH₃, (2) Cl/(3) C₂H₅, (2) Cl/(4) C₂H₅, (2) Cl/(5) C₂H₅, (2) Cl/(3) CF₃, (2) Cl/(4) CF₃, (2) Cl/(5) CF₃, (3) Cl/(4) CF₃, (2) Br/(3) F, (2) Br/(4) F, (2) Br/(5) F, (2) Br/(3) Cl, (2) Br/(4) Cl, (2) Br/(5) Cl, (2) Br/(3) CH₃, (2) Br/(4) CH₃, (2) Br/(5) CH₃, (2) Br/(3) CF₃, (2) Br/(4) CF₃, (2) Br/(5) CF₃, (2,3) (CH₃)₂, (2,4) (CH₃)₂, (2,5) (CH₃)₂, (3,4) (CH₃)₂, (2) CH₃/(3) F, (2) CH₃/(4) F, (2) CH₃/(5) F, (3) CH₃/(4) F, (2) CH₃/(3) Cl, (2) CH₃/(4) Cl, (2) CH₃/(5) Cl, (2) CH₃/(3) Br, (2) CH₃/(4) Br, (2) CH₃/(5) Br, (2) CH₃/(3) CF₃, (2) CH₃/(4) CF₃, (2) CH₃/(5) CF₃, (2) C₂H₅/(3) F, (2) C₂H₅/(4) F, (2) C₂H₅/(5) F, (2,3) (CF₃)₂, (2,4) (CF₃)₂, (2,5) (CF₃)₂, (2) CF₃/(3) F, (2) CF₃/(4) F, (2) CF₃/(5) F, (2) CF₃/(3) Cl, (2) CF₃/(4) Cl, (2) CF₃/(5) Cl, (2) Cl/(4) OCH₃, (2) Cl/(5) OCH₃, (2) F/(4) OCH₃, (2) OCF₃/(4) F, (2) OCF₃/ (4) Cl, (2) F/(4) OCF₃ ou (2) Cl/(4) OCF₃.

2. Composé de formule selon la revendication 1

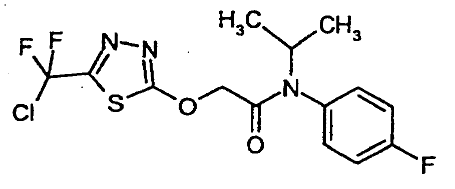

3. Procédé pour la préparation de composés selon la revendication 1, **caractérisé en ce qu'**on fait réagir le
5-chlorodifluorométhyl-2-méthylsulfonyl-1,3,4-thiadiazole de formule (II) 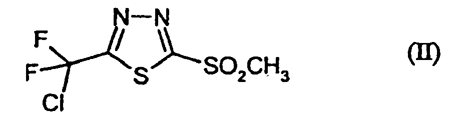 avec des hydroxyacétanilides de formule générale (III) 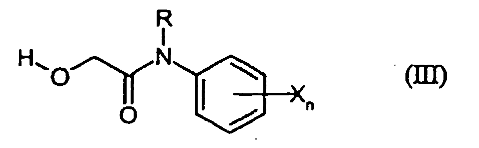 dans laquelle
n, R et X possèdent les significations données à la revendications 1,
éventuellement en présence d'un agent de liaison d'acide et éventuellement en présence d'un agent diluant.

4. Procédé pour la lutte contre une pousse de végétation indésirable, **caractérisé en ce qu'**on laisse agir au moins un composé selon l'une des revendications 1 à 2 sur les plantes indésirables et/ou leur espace vital.

5. Utilisation d'au moins un composé selon l'une des revendications 1 à 2 dans la lutte contre les plantes indésirables.

6. Agent herbicide **caractérisé par** une teneur en un composé selon l'une des revendication 1 à 2 et d'agents diluants et/ou agents de surface usuels.
